# EUROPEAN PATENT APPLICATION

(11) **EP 0 555 485 A1**
(43) Date of publication of application: **18.08.1993**
(21) Application number: 92915865.7
(22) Date of filing: 17.07.1992
(51) Int. Cl.: C07D 305/14, C12P 17/02, A61K 35/78, A61K 31/335

(54) **ANTITUMOR COMPOUND NSC-LSC1 AND PRODUCTION THEREOF**

(30) Priority: 17.07.1991 JP 176902/91
(71) Applicant: NIPPON STEEL CORPORATION, Tokyo 100-71 (JP)
(72) Inventor: SAITO, Koji, Nippon Steel Corp. Advanced Materials, Kawasaki-shi, Kanagawa 211 (JP); OHASHI, Hiroaki, Nippon Steel Corporation Advanced, Kawasaki-shi, Kanagawa 211 na (JP); TAHARA, Makoto, Nippon Steel Corporation Advanced, Kawasaki-shi, Kanagawa 211 (JP); SAKAMOTO, Tetsuo, Nippon Steel Corpor. Advanced, Kawasaki-shi, Kanagawa 211 (JP); HIBI, Masaaki, Nippon Steel Corporation Advanced, Kawasaki-shi, Kanagawa 211 (JP)
(74) Representative: Geering, Keith Edwin
(86) International application number: JP9200917
(87) International publication number: WO9302067

(57) **Abstract**

A taxol analog represented by estimated structural formula (I). It is one of the structural isomers of taxol and intensely inhibits the growth of mouse and human cancer cells. The production process comprises tissue culture of albumens of a plant belonging to the genus *Taxus*.

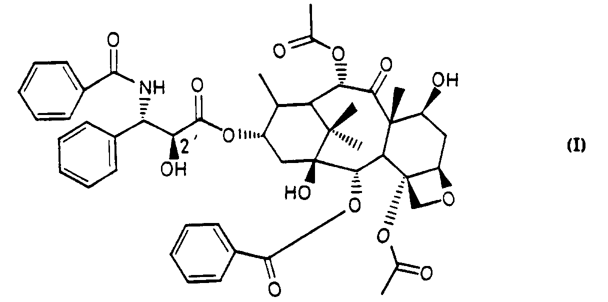

## Description

### TECHNICAL FIELD

The present invention relates to NSC-LSC1, which is a novel compound having an antitumor activity belonging to an analogue of taxol, and a process for producing the same. As with the taxol, the novel compound of the present invention has a low toxicity and a high antitumor activity.

### BACKGROUND ART

A large number of compounds having an antitumor activity and derived from plants are known in the art, and some of them are actually utilized as an antitumor agent. The utilization thereof has become an important means for treatment of tumors.

In general, many compounds having an antitumor activity and derived from plants are highly toxic to the human body, and also with respect to compounds approved as a drug, the toxicity to the human body is a large obstacle to the use thereof. Under these circumstances, taxol, which is a diterpene of taxane series, has attracted attention as a compound having a low toxicity and a high antitumor activity against human tumors. Taxol, extracted from the bark of Taxus brevifolia NUTT. belonging to the genus Taxus which in turn belongs to the family Taxaceae, has been subjected to clinical tests as an antitumor agent (see, for example, David G.I. Kingston, Pharmac. Ther. Vol. 52, pp. 1-34, 1991). In recent years, the production of taxol by tissue culture of a particular tissue from plants has also been reported (see, for example, U.S. Patent No. 5,019,504).

However, the development of a compound having diverse activities would be desired for contribution to chemotherapy useful for diverse malignant tumors, particularly cancers.

Accordingly, an object of the present invention is to develop a novel compound having an antitumor activity, which is expected to have a low toxicity and a specific antitumor activity against human tumors, and to provide a process for producing the same, which enables such a novel compound to be stably supplied.

### DISCLOSURE OF THE INVENTION

The present inventors have aimed at a compound having an antitumor activity and contained in plants belonging to the family Taxaceae and have made extensive and intensive studies. As a result, the inventors have succeeded in isolating a novel compound from a callus of Taxus brevifolia NUTT., which is clearly distinguished from taxol in the steric structure thereof although it exhibits the same high antitumor activity as that of taxol. Further, it has been confirmed that this compound can be efficiently produced from cali which can be easily induced and proliferated by tissue culture wherein female gametophytes of Taxus brevifolia NUTT is used as an explant. This has enabled the novel compound of the present invention to be stably supplied.

Namely, according to the present invention, there is provided a novel compound having an antitumor activity and identified by the following physicochemical properties:
① form: white powder
② molecular weight: m/e = 854 (MH⁺ as measured by FAB mass spectrum (NBA matrix)
③ elementary analysis (found):
   carbon 66.1%, hydrogen 6.0%
   oxygen 26.3%, nitrogen 1.6%
④ molecular formula: C₄₇H₅₁NO₁₄
⑤ specific rotation: [α]²⁴ = -49.7°
   [c = 0.36; methanol]
⑥ melting point: 213 - 216°C
⑦ solubility: sparingly soluble in water; easily soluble in methanol
⑧ proton nuclear magnetic resonance spectrum
   (¹H-NMR) (400 MHz; deuterated chloroform; chemical shift, ppm; coupling constant, Hz)
   1.145 (s, 3H); 1.242 (s, 3H);
   1.687 (s, 3H); 1.798 (s, 3H);
   1.82 (broad s, 1H); 1.88 (broad s, 1H);
   2.238 (s, 3H); 2.35 (m, 2H);
   2.385 (s, 3H); 2.55 (m, 2H);]
   3.56 (broad, 1H); 3.79 (d, j=6.8, 1H);
   4.18 (d, j=7.4, 1H); 4.31 (d, j=8.3, 1H);
   4.4 (m, 1H); 4.79 (d, j=2.4, 1H);
   4.95 (dd, j=1.8 and 7.2, 1H);
   5.67 (d, j=7.3, 1H);
   5.79 (dd, j=2.5 and 8, 1H);
   6.23 (t, j=8.5, 1H); 6.272 (s, 1H);
   6.99 (d, j=8.8, 1H); 7.39 (m, 4H);
   7.48 (m, 5H); 7.49 (m, 4H);
   7.73 (d, j=7.5, 1H); 8.13 (d, j=8.3, 1H).

For example, according to ¹H-NMR spectrum among the above-described physicochemical properties, a peak at 3.56 ppm is broad, and the correlation of the peak with a peak at 4.79 ppm is lost, so that is apparent that as represented by the following estimated structural formula, the compound of the present invention is different from taxol at least in the bonding form of a hydroxyl group at the 2'-position and is therefore structurally isomeric with taxol. 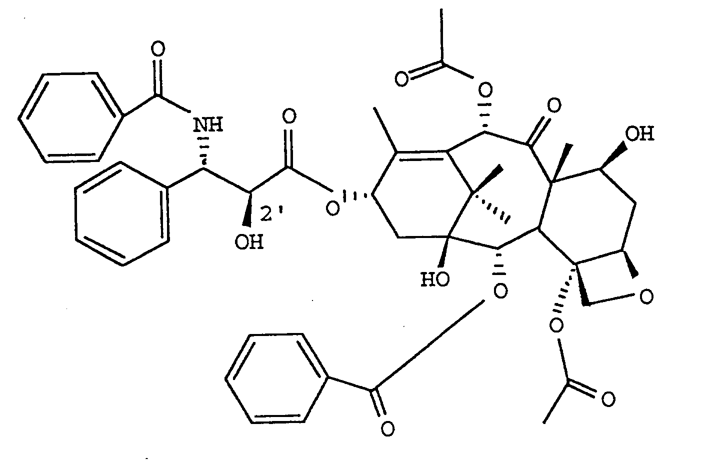
The present inventors have designated this compound as an antitumor compound NSC-LSC1 (hereinafter abbreviated "NSC-LSC1").

Further, according to another aspect of the present invention, there is provided a process for producing NSC-LSC1, which comprises steps of culturing a callus derived from female gametophytes of Taxus brevifolia NUTT., and extracting NSC-LSC1 from the resultant cultured product.

### BEST MODE FOR CARRYING OUT THE INVENTION

The estimated structural formula of NSC-LSC1 specified by the above-described physicochemical properties is supported also by the following ¹³C nuclear magnetic resonance spectrum (¹³C-NMR).

¹³C-NMR (400 MHz, deuterated chloroform, chemical shift, ppm):

| | | |
|---|---|---|
| 9.58 | 72.2 | 167.11 |
| 14.85 | 72.43 | 167.77 |
| 20.85 | 73.21 | 170.38 |
| 21.84 | 74.97 | 171.27 |
| 22.63 | 75.59 | 172.76 |
| 26.89 | 76.54 | 203.65 |
| 35.63 | 77.63 | |
| 35.72 | 79.08 | |
| 43.22 | 81.19 | |
| 45.65 | 84.43 | |
| 55.06 | 125-135 | |
| 58.65 | 131.25 | |

NSC-LSC1 provided by the present invention exhibits a high proliferation inhibitory activity against human or animal cancer cells transplanted into a test animal and further, a low toxicity to the animal which renders NSC-LSC1 promising as a compound for use in the field of chemotherapy of cancers.

The results of tests carried out at Institute of Applied Microbiology in the University of Tokyo, on proliferation inhibitory activity (IC₅₀) against P388 (mouse leukemia) and KB cells (human squamous cell carcinoma) are provided in Table 1. The IC₅₀ value is expressed in terms of the concentration of a drug required to reduce the cell proliferation by 50% to a control group (for reference, it is noted that in the antitumor effect evaluation test carried out at the National Cancer Institute (NCI) of the National Institute of Health in the U.S.A., substances having an IC₅₀ value against KB cells of 4000 ng/ml or less for synthetic substances and 20000 ng/ml or less for naturally occurring substances are defined as an effective antitumor substance.)

**Table**

| Inhibitory Activity of NSC-LSC1 against Proliferation of Mouse and Human Cancer Cells | |
|---|---|
| Test Cells | IC₅₀ (ng/ml) |
| · P388 | 3.3 |
| · KB cells | 19 |

As is apparent from the above-described results, NSC-LSC1 exhibits a very high antitumor activity against the human tumor cells.

NSC-LSC1 of the present invention can be advantageously produced by the process of the present invention which will now be described.

Specifically, according to the present invention, there is provided a process for producing a compound NSC-LSC1 by culturing tissues originating from Taxus brevifolia NUTT., which comprises:
a) a step of preparing an explant from living female gametophytes;
b) culturing the tissue prepared in the step (a) in a nutrient medium suitable for the induction of a callus to induce a callus;
c) culturing the callus obtained in the step (b) in a nutrient medium suitable for the proliferation of the callus; and
d) harvesting NSC-LSC1 from the resultant cultured product in the step c).

Examples of the preparation of an explant from living female gametophytes involve steps in which seeds collected from Taxus brevifolia NUTT. are sterilized with 70% ethanol and an aqueous solution of sodium hypochlorite, and the seeds are then aseptically cut open to remove female gametophytes, and thereafter, the female gametophytes are planted on a medium solidified by agar or Gelrite® (gellan gum manufactured by Merck & Co., Inc.) suitable for inducing a callus as described hereinbelow.

The living tissues obtained in the above-described steps are then cultured on a nutrient medium suitable for the induction of a callus and the induced callus is cultured in a nutrient medium suitable for the proliferation of callus cells. The primary components to be used in the nutrient medium include water; mineral nutrients, e.g., nitrogen (ammonium salts and nitrates), phosphorus, potassium, calcium, magnesium, sulfur, etc.; sugars, e.g., sucrose, glucose, fructose, maltose, etc.; minor amounts of inorganic nutrients; organic substances such as vitamins and amino acids; naturally originating substances such as coconut milk; and optionally, agar, Gelrite®, alginic acid, and agarose. The basic formulations of mineral nutrients used as basic media include Schenk & Hildebrandt medium (hereinafter referred to "SH medium"), Murashige & Skoog medium (hereinafter referred to as "MS medium"), Gamborg's B5 medium, White's medium, Nitsch & Nitsch medium, Nagata & Takebe medium and Woody Plant medium. To these basic media are further optionally added, as plant growth regulators, auxins, for example, 1-naphthaleneacetic acid (hereinafter referred to as "NAA"), indole-3-acetic acid (hereinafter referred to as "IAA"), indole-3-butyric acid (hereinafter referred to as "IBA"), and 2,4-dichlorophenoxyacetic acid (hereinafter referred to as "2,4-D"; cytokinins, for example, benzyladenine (hereinafter referred to as "BA"), kinetin, zeatin, and 6-furfurylaminopurine; and gibberellins (hereinafter referred to as "GA", for example, GA₁ and GA₃). Media comprising the above described basic media and, added thereto, auxins, cytokinins and gibberellins are preferable as the nutrient media suitable for inducing the callus according to the present invention. More specifically, a medium comprising the SH medium as the basic medium and, added thereto, BA as the cytokinin and GA₃ as the gibberellin, or a medium comprising the SH medium as the basic medium and, added thereto, NAA as the auxin and kinetin as the cytokinin is preferable. On the other hand, the nutrient media suitable for proliferating the callus cells preferably comprises a medium comprised of the above-described basic medium and, added thereto, cytokinins and auxins, more specifically a medium comprising the SH medium as the basic medium and, added thereto, kinetin as the cytokinin and NAA or 2,4-D as the auxin. The pH level of such media is adjusted to 5 to 7, preferably 5.5 to 6.0 with an appropriate acid or alkali.

The above-described cultures using these media can be carried out at a temperature in the range of 15-25°C with or without light-irradiation. In particular, the cultures for the proliferation of the callus cells are preferably carried out in a liquid medium by making use of a shaker.

The isolation of NSC-LSC1 from the cultured products thus obtained can be carried out according to the method of isolating various alkaloids from cultured tissue followed by purification, which is known per se. The term "cultured products" used herein is intended to include cultured cells and clumps (cali) of cells and their suspensions particularly when use is made of liquid media. Examples of the method of extracting NSC-LSC1 from the cultured product includes, but is not limited to, a general method which comprises separating cali from media, drying and pulverizing the same, subjecting the resulting powder to extraction with an appropriate organic solvent, optionally washing the organic phase with water, drying the organic phase, and then removing the solvent by distillation. If necessary, the extracted NSC-LSC1 can be purified by various chromatographic treatments or recrystallization, etc. Examples of the organic solvent which can be used in the extraction include chlorinated hydrocarbons such as methylene chloride and dichloroethane, alcohols such as methanol, ethanol and isopropyl alcohol, and mixtures of the above organic solvents.

The present invention will now be described in greater detail by referring to the following working examples, to which the present invention is not restricted.

### EXAMPLE

A seed of Taxus brevifolia NUTT. (a native tree which grows at Fish Lake, Oregon, U.S.A.) from which aril had been removed and stored in a refigerator was immersed in an aqueous solution of 70% W/W ethanol for one minute, and then in an aqueous sodium hypochlorite solution having an effective chlorine concentration of 1% for 20 minutes to sterilize the seed, and thereafter, was rinsed three times with sterilized water. After the sterilization, the hard external seed coat and internal seed coat were removed with a knife and tweezers, and the resulting embryos and female gametophytes were then separated from each other.

The female gametophytes thus prepared were planted on a SH medium (supplemented with 0.25% of Gelrite to provide a solid medium) prepared by adding two plant growth regulators, benzyladenine (BA) and GA3, which is a kind of gibberellin, and adjusting the pH value to 5.8. The concentrations of BA and GA3 were 1, 5 or 10 mg/l and 0, 1, 10 or 100 mg/l, respectively. The planted female gametophytes were cultured at a temperature of 20°C or 25°C under a light (for 16 hr per day). As a result, it was found that a callus was induced from the gametophytes after approximately 2 months of the initiation of the culture.

The callus thus induced was subcultured in a SH medium (a liquid medium) supplemented with naphthaleneacetic acid (NAA) and kinetin and by adjusting the pH value to 5.8. The concentrations of NAA and kinetin added to the medium were 5 mg/l and 0, 0.1 or 0.5 mg/l, respectively. The amount of the medium was 40 ml per Erlenmyer flask having a capacity of 100 ml, and suspension culture was conducted at 100 rpm by using a flask shaker. The culture was conducted at a temperature of 20°C under a lighting condition darkness.

Four weeks after the initiation of the culture, the proliferation of the callus was examined. As a result, with respect to the medium to which NAA alone had been added to a concentration of 5 mg/l, the proliferation rate was about 5 times that of the callus planted in the above-described liquid medium. Further, with respect to the medium to which NAA alone had been added to a concentration of 5 mg/l, the culture was conducted at a temperature of 20°C or 25°C and under darkness or illumination (for 16 hr per day) for 4 weeks. As a result, proliferation was observed in every combination, however, the best result was obtained particularly when the culture was conducted at 20°C in darkness. Further, when 2,4-D was used instead of NAA as the auxins, proliferation similar to that in the case where use was made of NAA was observed.

The callus thus proliferated was harvested and dried. The dried callus of 30g was throughly homogenized in a mortar, combined with 500 ml of a mixed solution comprising methanol and methylene chloride in a ratio of 1 : 1, and shaking and extraction were conducted at room temperature for 16 hr in a Sakaguchi flask. This procedure was repeated three times, and the extract was concentrated in vacuo to provide 1.03g of a brown substance. The brown substance was subjected to partitioning with 500 ml of a mixed solution comprising methylene chloride and water in a ratio of 1 : 1 to recover the methylene chloride layer. The above procedure was repeated several times, and the extract was concentrated in vacuo to provide 530 mg of a brown substance. The resultant compound was dissolved in methanol and fractionated by high performance liquid chromatography [column: Dynamax (trademark), 60A, 8 µm, C18, 4.6 mm* 250 mm (manufactured by Rainin Instrument Company Inc., U.S.A.); composition of mobile phase: comprising methanol, water and acetonitrile in a ratio of 20 : 41 : 39].

Among the resultant fractions, a fraction considered to have activity was concentrated in vacuo. This procedure was repeated to provide 11 mg of a white powdery substance. This powder was dissolved in methanol and subjected to high performance liquid chromatography [column: Dynamax (trademark), 60A, 8 µm, C18-phenyl 4.6 mm* 250 mm (manufactured by Rainin Instrument Company Inc., U.S.A.); composition of mobile phase: comprising methanol, water and acetonitrile in a ratio of 20 : 45 : 35; detecting light wavelength: 227 nm] to determine the absorption of the detecting light wavelength to the retention time of the column. As a result, only one peak was observed, that is, it was found that a compound having a very high purity was provided.

The high-purity compound was found to have the above-described physicochemical properties and antitumor activity.

### INDUSTRIAL APPLICABILITY

The NSC-LSC1 of the present invention has a low toxicity and a high antitumor activity and can be stably supplied by tissue culture wherein use is made of female gametophytes of Taxus brevifolia NUTT., which renders the present invention utilizable in the manufacture of pharmaceuticals.

## Claims

1. A compound NSC-LSC1 analogous to taxol and identified by the following physicochemical properties:
① form: white powder
② molecular weight: m/e = 854 (MH⁺) as measured by FAB mass spectrum (NBA matrix)
③ elementary analysis (found):
carbon 66.1%, hydrogen 6.0%
oxygen 26.3%, nitrogen 1.6%
④ molecular formula: C₄₇H₅₁NO₁₄
⑤ specific rotation: [α]²⁴ = -49.7°
(c = 0.36; methanol)
⑥ melting point: 213 - 216°C
⑦ solubility: sparingly soluble in water; easily soluble in methanol
⑧ proton nuclear magnetic resonance spectrum
(¹H-NMR) (400 MHz; CDCl₃, ppm)
1.145 (s, 3H); 1.242 (s, 3H);
1.687 (s, 3H); 1.798 (s, 3H);
1.82 (broad s, 1H); 1.88 (broad s, 1H);
2.238 (s, 3H); 2.35 (m, 2H);
2.385 (s, 3H); 2.55 (m, 2H);
3.56 (broad, 1H); 3.79 (d, j=6.8, 1H);
4.18 (d, j=7.4, 1H); 4.31 (d, j=8.3, 1H);
4.4 (m, 1H); 4.79 (d, j=2.4, 1H);
4.95 (dd, j=1.8 and 7.2, 1H);
5.67 (d, j=7.3, 1H);
5.79 (dd, j=2.5 and 8, 1H);
6.23 (t, j=8.5, 1H); 6.272 (s, 1H);
6.99 (d, j=8.8, 1H); 7.39 (m, 4H);
7.48 (m, 5H); 7.49 (m, 4H);
7.73 (d, j=7.5, 1H); 8.13 (d, j=8.3, 1H).

2. A process for producing a compound NSC-LSC1 according to claim 1 by culturing tissues originating from Taxus brevifolia NUTT, which comprises:
a) a step of preparing an explant from living female gametophytes;
b) culturing the tissue prepared in the step (a) in a nutrient medium suitable for the induction of a callus;
c) culturing the callus obtained in the step (b) in a nutrient medium suitable for the proliferation of the callus; and
d) harvesting NSC-LSC1 from the resultant cultured product in the step c).
